# EUROPEAN PATENT APPLICATION

(11) **EP 4 510 398 A2**
(43) Date of publication of application: **19.02.2025**
(21) Application number: 24193472.8
(22) Date of filing: 08.08.2024
(51) Int. Cl.: H01S 5/11, H01S 5/183, H01S 5/20

(54) **PCSEL FOR GLUCOSE MONITORING**

(30) Priority: 15.08.2023 US 202363519805 P; 11.06.2024 US 202418739612
(71) Applicant: II-VI Delaware, Inc., Wilmington, DE 19890 (US)
(72) Inventor: ENG, Julie, Wilmington, 19890 (US); KOCOT, Chris, Wilmington, 19890 (US); TATARCZAK, Anna, Wilmington, 19890 (US); CHEN, Young-Kai, Wilmington, 19890 (US)
(74) Representative: Schmidt, Christian

(57) **Abstract**

This disclosure describes a glucose monitor with one or more photonic crystal surface-emitting lasers (PCSELs). The PCSEL comprises a plurality of photonics crystal sections. Each photonics crystal section is operable to emit light of a different wavelength. The horizontal emission by each photonics crystal section may be constrained by edge lattice sections.

## Description

### CROSS REFERENCE TO RELATED APPLICATIONS

This application claims the benefit of and priority to U.S. Provisional Patent Application No. 63/519,805, titled "PCSEL FOR GLUCOSE MONITORING," filed August 15, 2023, the disclosure of which is incorporated herein by reference in its entirety.

### BACKGROUND

Limitations and disadvantages of traditional systems and methods for glucose monitoring will become apparent to one of skill in the art, through comparison of such approaches with some aspects of the present method and system set forth in the remainder of this disclosure with reference to the drawings.

### BRIEF SUMMARY

The systems and methods disclosed herein use a Photonic Crystal Surface-Emitting Laser (PCSEL) to monitor glucose, substantially as illustrated by and/or described in connection with at least one of the figures, as set forth more completely in the claims.

### BRIEF DESCRIPTION OF THE DRAWINGS

FIG. 1 illustrates a first example top emitter structure with a PCSEL, in accordance with various example implementations of this disclosure.
FIG. 2 illustrates an example bottom emitter structure with a PCSEL, in accordance with various example implementations of this disclosure.
FIG. 3 illustrates a second example top emitter structure with a PCSEL, in accordance with various example implementations of this disclosure.
FIG. 4 illustrates a third example top emitter structure with a PCSEL, in accordance with various example implementations of this disclosure.
FIG. 5 illustrates an example structure that co-integrates two monolithic arrays, with different ranges of wavelengths, on the same substrate, in accordance with various example implementations of this disclosure.

### DETAILED DESCRIPTION

Millions of people have diabetes and require glucose level monitoring multiple times a day. However, monitoring systems that are available today are invasive. While monitoring systems today may use lasers, these systems are not adequate. For example, known existing solutions with a 2.0-2.4 µm laser source include bulk lasers (such as an excimer laser) that are not applicable for the compact wearable glucose sensor application. Another existing solution includes distributed-feedback laser (DFB) lasers. In a glucose level monitoring application with DFB lasers, the epi stack for each wavelength is different and the DFB lasers must be integrated on the same platform. This integration limits the number of wavelengths one can use with high yield, which also makes such a solution expensive. Additionally, existing solutions have a relatively broad spectral bandwidth (SBW) for each wavelength (e.g., ~1 nm) and a low beam quality (e.g., M² > 1.1). While tunable lasers are also compatible with the 2.0-2.4 µm range emission, precise wavelength tuning of the laser is very difficult.

This disclosure describes a non-invasive system and method to monitor glucose. As described below, in accordance with various example implementations of this disclosure, a 2.0-2.4 µm Photonic Crystal Surface-Emitting Laser (PCSEL) may be used for non-invasive glucose monitoring and other medical optical sensing applications. The PCSEL provides superior beam purity and superior beam quality for such optical sensing applications. Additionally, the PCSEL enables a monolithic integration for a range of wavelengths that may be required for an absorption-based glucose sensing application.

The PCSEL's emission wavelength is defined through the photonic crystal lattice design and may be limited by the etching tool capability. The wavelength precision is significantly better than existing solutions. PCSELs have a significantly lower SBW (e.g., <0.1 nm) and a superior beam quality (e.g., M² < 1.1). PCSELs enable monolithic integration for a larger number of wavelengths. Therefore, a single epi design may be used for multiple wavelengths.

While this disclosure describes using monolithic PCSEL arrays for glucose monitoring with wavelengths between 2.0-2.4 µm, other wavelength ranges may also be supported by using a different material set for the active region. For example, for wavelengths between 2.0-2.4 µm, highly strained InGaAs and GaSb materials may be used for the active region as the gain of InGaAs and GaSb supports this wavelength range.

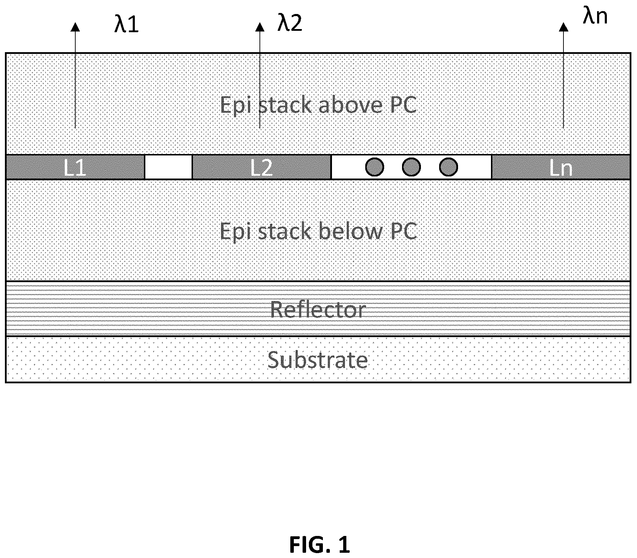

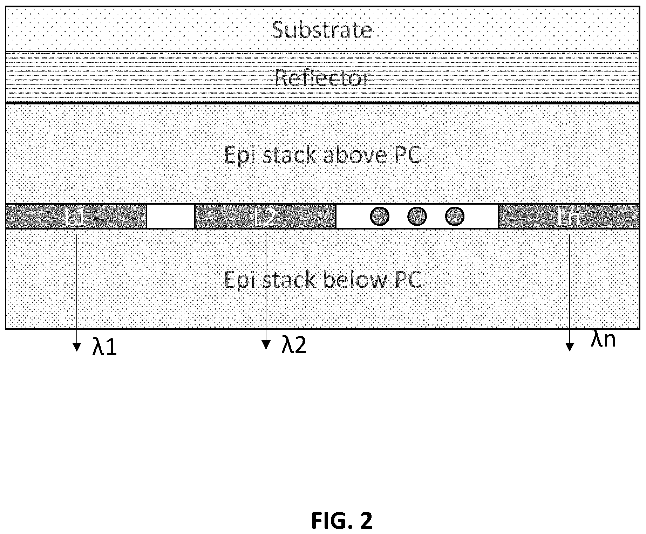

The PCSEL emission direction can be either top (FIGs. 1, 3, 4 and 5) or bottom (FIG. 2), depending on the application requirements.

FIG. 1 illustrates a first example top emitter structure with a PCSEL, in accordance with various example implementations of this disclosure.

The photonic crystal (PC) lattices L1, L2... Ln are designed to output wavelengths λ1, λ2... λn, respectively. For a large optical aperture (e.g., OA > 30 µm), the horizontal mode may be constrained by the lattice. The epi stacks (above and/or below the PC) may include a p-n junction.

FIG. 2 illustrates an example bottom emitter structure with a PCSEL, in accordance with various example implementations of this disclosure.

As in FIG. 1, the PC lattices L1, L2... Ln are designed to output wavelengths λ1, λ2... λn, respectively. For a large optical aperture (e.g., OA > 30 µm), the horizontal mode may be constrained by the lattice. The epi stack above the PC may comprise a top distributed Bragg reflector (DBR) that is 100% reflective. The epi stack below the PC may comprise a bottom DBR that is not fully reflective.

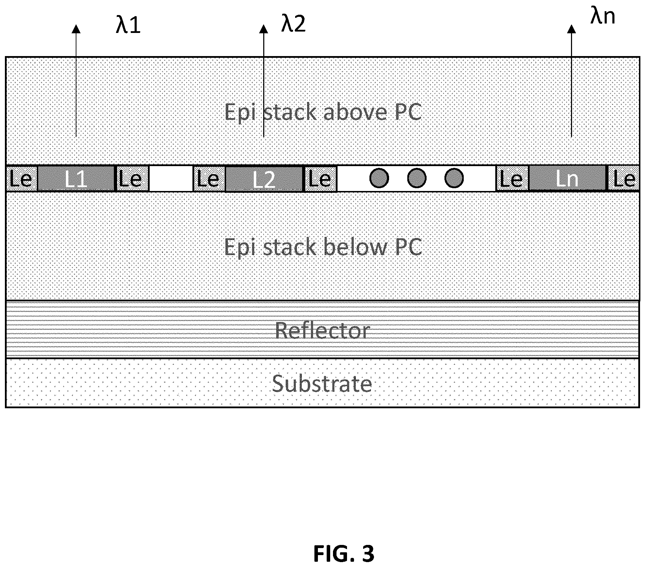

FIG. 3 illustrates a second example top emitter structure with a PCSEL, in accordance with various example implementations of this disclosure.

As in FIGs. 1 and 2, the PC lattices L1, L2... Ln are designed to output wavelengths λ1, λ2... λn, respectively. The horizontal mode, in FIG. 3, is constrained by the edge lattice (Le). The edge lattice allows for an OA < 30 µm. The edge lattice may also be used for smaller apertures in the bottom emitting configuration of FIG. 2.

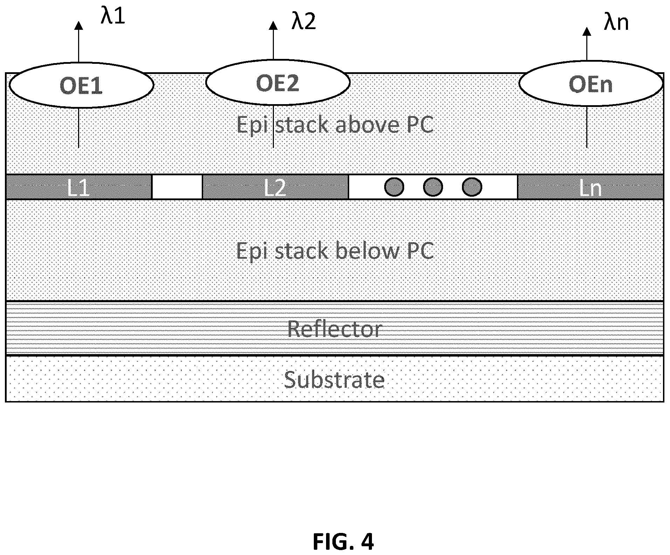

FIG. 4 illustrates a third example top emitter structure with a PCSEL, in accordance with various example implementations of this disclosure.

As in FIGs. 1-3, the PC lattices L1, L2... Ln are designed to output wavelengths λ1, λ2... λn, respectively. Optical elements (OE1, OE2, ..., OEn) are added in FIG. 4. The optical elements may comprise lenses and/or diffusers that may be placed (e.g., etched, deposited) on the emission surface for beam shaping purposes.

Horizontal modes in a PCSEL are constrained by the photonic crystal if the optical aperture is large (>30 µm), or by a secondary (outer) photonic crystal for smaller apertures (<30 µm). The photonic bandgap of the "outer" photonics crystal is smaller and hence it serves as a reflector.

For the GaSb in the active region, the epi stack for a p-n junction implementation consists of GaAs substrate and cladding layers. The photonic crystal layer can comprise GaAs or GaSb.

The vertical mirror stack may comprise GaAs and AlGaAs mirrors that cover the required wavelength range. Additional lattice matching layers or wafer bonding may be required for the GaSb active region due to a lattice mismatch.

For the solution with strained InGaAs in the active region, regrowth may be used to form the photonic crystal and epi layers above it. The epi stack for InGaAs in the active region may comprise an InP substrate and cladding layers, a reflector, and a GalnAsP photonic crystal.

For both GaSb and InGaAs solutions, at least two stack configurations are possible. One possible configuration comprises p on one side of the active region and n on the other. Another configuration comprises n on both sides of the active region and a tunnel junction (TJ) in between.

The PCSEL array can be manufactured monolithically for a range of wavelengths. The wavelength range in each monolithic array may depend on the uniformity requirements. The power uniformity across wavelengths depends on the gain peak of the active region material. The wavelength range may or may not cover the range between 2.0-2.4 µm. If the wavelength range does not cover the range between 2.0-2.4 µm, multiple, monolithic arrays may be used for the full range.

The wavelength of each device in an array may be different and controlled by lithography (photonic crystal design). In such a case, the lattice constant will be different for each device.

A vertical mirror stack may be shared between all the wavelengths or the subset of wavelengths depending on the uniformity requirements. The total response is a combination of the gain curve response and the mirror design and this feature may be used to design a "flat" response and hence a better uniformity.

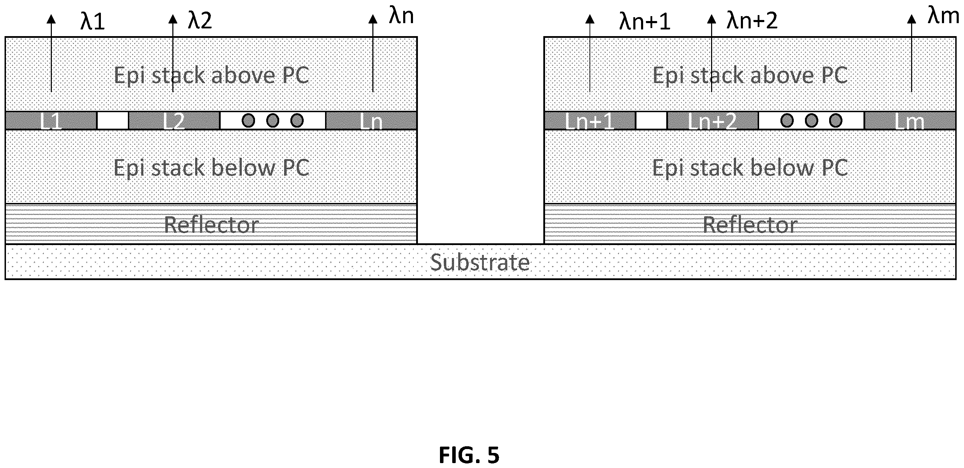

FIG. 5 illustrates an example structure that co-integrates two monolithic arrays, with different ranges of wavelengths, on the same substrate, in accordance with various example implementations of this disclosure.

Multiple monolithic arrays could be integrated on the same platform through for example a pick and place process. Each monolithic array can have a separately optimized DBR stack. Due to the low spectral bandwidth of the PCSEL and the high central wavelength design accuracy, the achievable wavelength spacing can be <5 nm.

Wavelengths may also be spaced over temperature. The central wavelength of each device may have the same thermal shift. The index of refraction may change with temperature. For example, the index of refraction may change by 0.07 nm for every degree Celsius. The relative wavelength spacing may be maintained over temperature. Therefore, the glucose monitoring application with PCSELs may not need cooling. The wavelength spacing may depend on the etching capability.

The monitoring system may be compatible with the integrated optics (i.e., deposited lenses and/or diffusers). The lasers in an array can be powered up simultaneously or sequentially. To support the sequential wavelength sweep, each laser can have an isolated metal contact and driver.

In the following, a set of aspects is disclosed. The aspects are numbered to facilitate referencing the features of one aspect in other aspects. The aspects form part of the disclosure of the present application and could be made subject to independent and/or dependent claims irrespective of what currently is claimed in the application. The aspects are:
1. A laser device, comprising:
   a first photonics crystal section operable to out-couple light, of a first wavelength, in a vertical direction; and
   a second photonics crystal section operable to out-couple light, of a second wavelength that is different than the first wavelength, in the vertical direction.
2. The laser device of aspect 1, wherein:
   the first photonics crystal section and the second photonics crystal section are located at a same fabrication layer.
3. The laser device of aspect 1, wherein the laser device comprises:
   a first epitaxy layer operably coupled below the first photonics crystal section and the second photonics crystal section, and
   a second epitaxy layer operably coupled below the first photonics crystal section and the second photonics crystal section.
4. The laser device of aspect 1, wherein a glucose monitor comprises the laser device.
5. The laser device of aspect 1, wherein the laser device is a bottom-emitting laser.
6. The laser device of aspect 1, wherein the laser device is a top-emitting laser.
7. The laser device of aspect 1, wherein the first wavelength and the second wavelength are between 2.0 and 2.4 µm.
8. The laser device of aspect 1, wherein:
   one or more edge lattice sections, adjacent to the first photonics crystal section, are operable to constrain a horizontal mode of the first wavelength, and
   one or more edge lattice sections, adjacent to the second photonics crystal section, are operable to constrain a horizontal mode of the second wavelength.
9. The laser device of aspect 1, wherein the laser device comprises:
   one or more optical elements placed on an emission surface.
10. The laser device of aspect 1, wherein:
   the first wavelength and the second wavelength are determined according to temperature.
11. A method of fabricating a laser device, comprising:
   fabricating a first photonics crystal section operable to out-couple light, of a first wavelength, in a vertical direction; and
   fabricating a second photonics crystal section operable to out-couple light, of a second wavelength that is different than the first wavelength, in the vertical direction.
12. The method of aspect 11, wherein:
   the first photonics crystal section and the second photonics crystal section are located at a same fabrication layer.
13. The method of aspect 11, wherein the method comprises:
   fabricating a first epitaxy layer operably prior to fabricating the first photonics crystal section and the second photonics crystal section, and
   fabricating a second epitaxy layer atop the first photonics crystal section and the second photonics crystal section.
14. The method of aspect 11, wherein a glucose monitor comprises the laser device.
15. The method of aspect 11, wherein the laser device is a bottom-emitting laser.
16. The method of aspect 11, wherein the laser device is a top-emitting laser.
17. The method of aspect 11, wherein the first wavelength and the second wavelength are between 2.0 and 2.4 µm.
18. The method of aspect 11, wherein the method comprises:
   fabricating one or more edge lattice sections, adjacent to the first photonics crystal section, are operable to constrain a horizontal mode of the first wavelength, and
   fabricating one or more edge lattice sections, adjacent to the second photonics crystal section, are operable to constrain a horizontal mode of the second wavelength.
19. The method of aspect 11, wherein the method comprises:
   placing one or more optical elements on an emission surface.
20. The method of aspect 11, wherein:
   the first wavelength and the second wavelength are determined according to temperature.

Implementations as described herein may relate to the following: A glucose monitor with one or more photonic crystal surface-emitting lasers (PCSELs). The PCSEL comprises a plurality of photonics crystal sections. Each photonics crystal section is operable to emit light of a different wavelength. The horizontal emission by each photonics crystal section may be constrained by edge lattice sections.

The present application claims priority of U.S. Provisional Patent Application No. 63/519,805, filed August 15, 2023, and of U.S. Patent Application No. 18/739,612, filed June 11, 2024. The entire disclosure of these applications is hereby explicitly incorporated by reference into the present application.

As used herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As used herein, "and/or" means any one or more of the items in the list joined by "and/or". As an example, "x and/or y" means any element of the three-element set {(x), (y), (x, y)}. As another example, "x, y, and/or z" means any element of the seven-element set {(x), (y), (z), (x, y), (x, z), (y, z), (x, y, z)}. As used herein, the term "exemplary" means serving as a non-limiting example, instance, or illustration. As used herein, the terms "e.g.," and "for example" set off lists of one or more non-limiting examples, instances, or illustrations. As used herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.). As used herein, the term "based on" means "based at least in part on." For example, "x based on y" means that "x" is based at least in part on "y" (and may also be based on z, for example).

While the present method and/or system has been described with reference to certain implementations, it will be understood by those skilled in the art that various changes may be made and equivalents may be substituted without departing from the scope of the present method and/or system. In addition, many modifications may be made to adapt a particular situation or material to the teachings of the present disclosure without departing from its scope. Therefore, it is intended that the present method and/or system not be limited to the particular implementations disclosed, but that the present method and/or system will include all implementations falling within the scope of the appended claims.

## Claims

1. A laser device, comprising:
a first photonics crystal section operable to out-couple light, of a first wavelength, in a vertical direction; and
a second photonics crystal section operable to out-couple light, of a second wavelength that is different than the first wavelength, in the vertical direction.

2. The laser device of claim 1, wherein:
the first photonics crystal section and the second photonics crystal section are located at a same fabrication layer.

3. The laser device of any one of the preceding claims, wherein the laser device comprises:
a first epitaxy layer operably coupled below the first photonics crystal section and the second photonics crystal section, and
a second epitaxy layer operably coupled below the first photonics crystal section and the second photonics crystal section.

4. The laser device of any one of the preceding claims, wherein a glucose monitor comprises the laser device.

5. The laser device of any one of the preceding claims, wherein the laser device is a bottom-emitting laser or a top-emitting laser.

6. The laser device of any one of the preceding claims, wherein the first wavelength and the second wavelength are between 2.0 and 2.4 µm.

7. The laser device of any one of the preceding claims, wherein:
one or more edge lattice sections, adjacent to the first photonics crystal section, are operable to constrain a horizontal mode of the first wavelength, and
one or more edge lattice sections, adjacent to the second photonics crystal section, are operable to constrain a horizontal mode of the second wavelength.

8. The laser device of any one of the preceding claims, wherein the laser device comprises:
one or more optical elements placed on an emission surface, and/or, wherein
the first wavelength and the second wavelength are determined according to temperature.

9. A method of fabricating a laser device, comprising:
fabricating a first photonics crystal section operable to out-couple light, of a first wavelength, in a vertical direction; and
fabricating a second photonics crystal section operable to out-couple light, of a second wavelength that is different than the first wavelength, in the vertical direction.

10. The method of claim 9, wherein:
the first photonics crystal section and the second photonics crystal section are located at a same fabrication layer.

11. The method of any one of the preceding claims 9 to 10, wherein the method comprises:
fabricating a first epitaxy layer operably prior to fabricating the first photonics crystal section and the second photonics crystal section, and
fabricating a second epitaxy layer atop the first photonics crystal section and the second photonics crystal section.

12. The method of any one of the preceding claims 9 to 11, wherein a glucose monitor comprises the laser device.

13. The method of any one of the preceding claims 9 to 11, wherein the laser device is a bottom-emitting laser or a top-emitting laser.

14. The method of any one of the preceding claims 9 to 13, wherein the first wavelength and the second wavelength are between 2.0 and 2.4 µm.

15. The method of any one of the preceding claims 9 to 14, wherein the method comprises:
fabricating one or more edge lattice sections, adjacent to the first photonics crystal section, are operable to constrain a horizontal mode of the first wavelength, and
fabricating one or more edge lattice sections, adjacent to the second photonics crystal section, are operable to constrain a horizontal mode of the second wavelength, wherein the method optionally further comprises:
i) placing one or more optical elements on an emission surface, and/or wherein
ii) the first wavelength and the second wavelength are determined according to temperature.
